# EUROPEAN PATENT SPECIFICATION

(11) **EP 3 691 587 B1**
(45) Date of publication and mention of the grant of the patent: **22.05.2024**
(21) Application number: 18864336.5
(22) Date of filing: 01.10.2018
(51) Int. Cl.: A61F 13/02, A61M 25/02, A61F 13/00

(54) **REINFORCED WINDOW DRESSING**
VERSTÄRKTER FENSTERVERBAND
PANSEMENT À FENÊTRE RENFORCÉ

(30) Priority: 02.10.2017 US 201762566890 P
(43) Date of publication of application: 12.08.2020
(73) Proprietor: Medline Industries, LP, Northfield, IL 60093 (US)
(72) Inventor: STANKIEWICZ, Michael, J., Chicago, IL 60640 (US); NOSKOWICZ, David, S., Spring Grove, IL 60081 (US)
(74) Representative: Gaunt, Thomas Derrick
(86) International application number: PCT/US2018/053808
(87) International publication number: WO 2019/070610

(56) References cited:
- WO-A1-2015/130608
- US-A1- 2008 132 821
- US-A1- 2010 198 161
- US-A1- 2011 166 492
- US-A1- 2014 005 607
- US-A1- 2014 005 607
- US-A1- 2016 193 452
- US-A1- 2017 232 226
- US-B1- 7 294 752
- US-B1- 7 294 752
- US-B1- 8 486 004
- US-B1- 9 566 417
- US-B2- 8 212 101
- US-B2- 9 220 869
- US-B2- 9 457 123

## Description

### FIELD OF THE INVENTION

The present invention relates generally to medical dressings, and in particular to window dressings for use in securing a catheter at an insertion site.

### BACKGROUND

In many instances, it is necessary to insert a catheter into a patients skin. Once the catheter is inserted, the catheter may exert pressure on the skin at the insertion site, or it may be necessary to constrain the catheter so that it does not catch on clothing or other hazards. Accordingly, a medical clinician (i.e., doctors, nurses, and other medical personnel) may apply strips of medical tape to attempt to secure the catheter or associated medical tubing. Another clinical practice is to suture a catheter hub to a patient's skin to roughly secure the catheter to the patient. Alternatively, it is known in the art relating to medical dressings to apply a dressing to a patient's skin to cover a catheter insertion site at which the catheter punctures a patient's skin. Further still, a variety of catheter and medical tubing securement devices are available for use in the medical field. These securement devices, however, are often bulky and cumbersome, hard to dress with a dressing, and may have costly and complex mechanical features.

It is also known in the medical field that poorly dressed and poorly secured catheters and associated tubing may lead to irritation of the insertion site, necessitating movement of the catheter to a new insertion site. In addition, poorly secured catheters are susceptible to accidental dislodgement from the insertion site. For example, medical tubing connected to indwelling catheters, infusion needles and the like may be subjected to inadvertent but significant pulling forces either caused directly by patient movement or by snagging of the tubing on other objects. These pulling forces peel the medical tape or dressing securing the catheter and/or tubing off the patient's skin. This exposes the catheter, infusion needle, etc. to movement inward or outward, increasing the likelihood that the catheter, infusion needle, etc. will fail and have to be replaced and inserted into a new insertion site. Also, this may weaken the adhesion between the dressing and the patient's skin, potentially exposing the insertion site to harmful bacteria.

See, e.g., US8486004 B1, US9566417 B1, WO2015130608 A1, US2016/193452 A1.

Accordingly, there is a need for an improved dressing that addresses these and other issues known in the prior art. In particular, there is needed a dressing having an improved structure for securing the catheter and associated tubing or other accessories.
The invention is set out in the appended set of claims.

### BRIEF DESCRIPTION OF THE DRAWINGS

Advantages of the invention will become apparent upon reading the following detailed description and upon reference to the drawings.
FIG. 1 is a top plan view of a window dressing according to one example not being part of the invention.
FIG. 2 is an exploded cross-sectional view taken along a longitudinal centerline of the window dressing of FIG. 1.
FIG. 3 is an exploded cross-sectional view taken along a longitudinal centerline of a further example not being part of the invention.
FIG. 4 is a cross-sectional view taken along a longitudinal centerline of the window dressing of FIG. 3 in an assembled state prior to application on a patient.
FIG. 5 is a top plan view of a window dressing according to a further example not being part of the invention.
FIG. 6 is a top plan view of a window dressing according to a further example not being part of the invention.
FIG. 7 is a top plan view of a window dressing according to a further example not being part of the invention.
FIG. 8 is a top plan view of a window dressing according to an embodiment of the invention.

While the invention is susceptible to various modifications and alternative forms, embodiments have been shown by way of example in the drawings and will be described in detail herein. It should be understood, however, that the invention is not intended to be limited to the particular forms disclosed. Rather, the invention is to cover all modifications, and alternatives falling within the scope of the claims

### DESCRIPTION

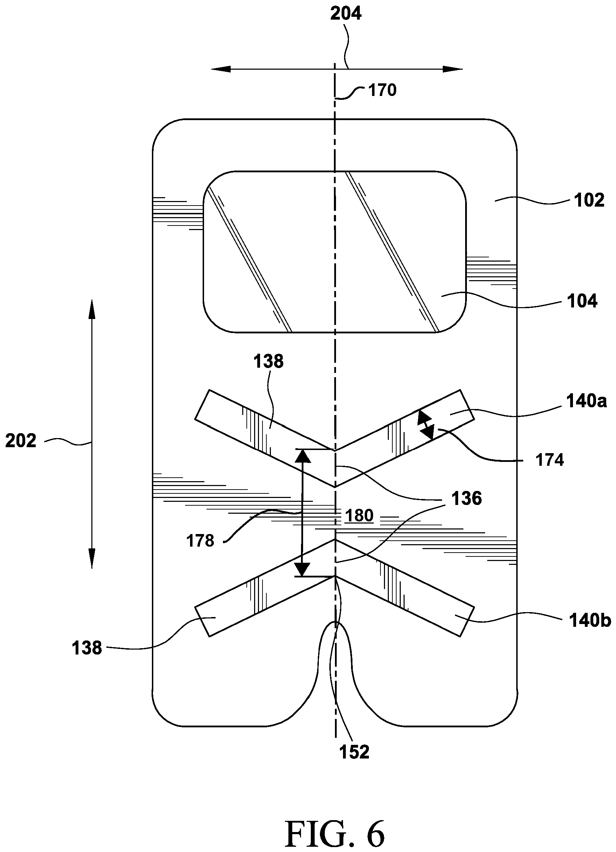

Referring to the drawings, like numbers indicate like parts throughout the views. As used in the description herein and throughout the claims, the following terms take the meanings explicitly associated herein, unless the context clearly dictates otherwise: the meaning of "a," "an," and "the" includes plural reference, the meaning of "in" includes "in" and "on." Relational terms such as first and second, top and bottom, forward and rearward, and the like may be used solely to distinguish one entity or action from another entity or action without necessarily requiring or implying any actual such relationship, direction or order between such entities or actions. Reference may also be made to longitudinal and latitudinal directions in reference to the window dressing described herein. As shown in in FIG. 6, longitudinal refers to the direction along a major axis of the dressing as illustrated by arrow 202, and latitudinal refers to the direction along a minor axis perpendicular to the major axis as illustrated by arrow 204.

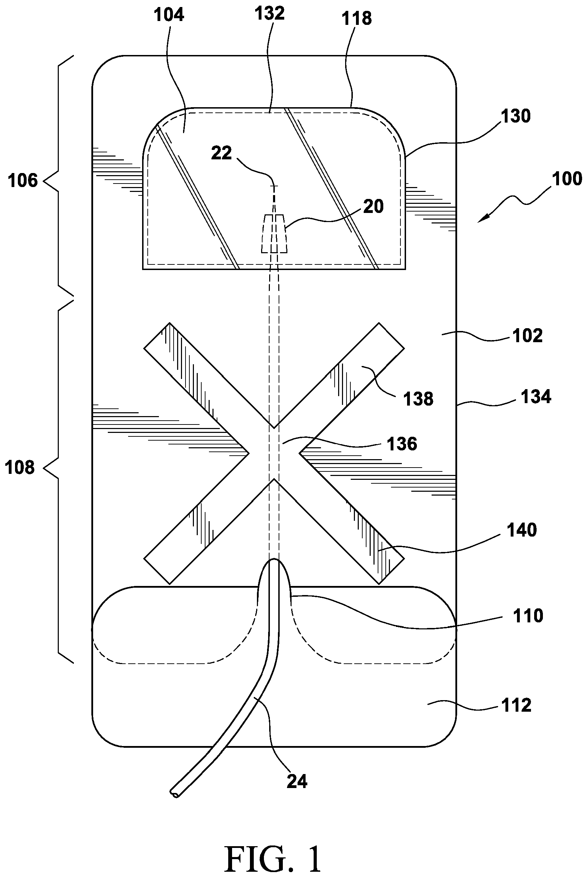

As illustrated in FIG. 1, a window dressing 100 may be used to secure a catheter 20 inserted at a catheter insertion site 22. Such insertion sites may include a PICC ("peripherally inserted central venous catheter") insertion site, a jugular insertion site, a subclavian insertion site, a femoral insertion site, or an implanted port insertion site. The window dressing 100 is capable of securing a variety of sizes, shapes, and types of catheters (single lumen, double lumen, triple, and quad lumen), infusion needles, and associated hubs, ports, and tubing. The window dressing 100 may provide protection against microbial ingress and site or patient systemic infection and may secure a catheter and associated hubs, ports, and tubing so that forces acting on the tubing and catheter do not peel the dressing from a patient's skin or cause the catheter to become dislodged.

As shown in FIGS. 1-2, the dressing 100 may comprise a primary layer 102. The primary layer may comprise a fabric material that may be a woven or nonwoven fabric. The fabric material may be formed from an appropriate material including manmade or naturally occurring fibers. Alternatively, the primary layer 102 may comprise a polymeric film. In various embodiments, the film or fabric layer may comprise plastic (PVC, polyethylene or polyurethane), latex, tape, cloth, paper or other materials. Still further, the primary layer may comprise a combination of layers including fabrics or films or as well as coatings or additional supplemental materials. For example, the primary layer may comprise materials having antimicrobial properties.

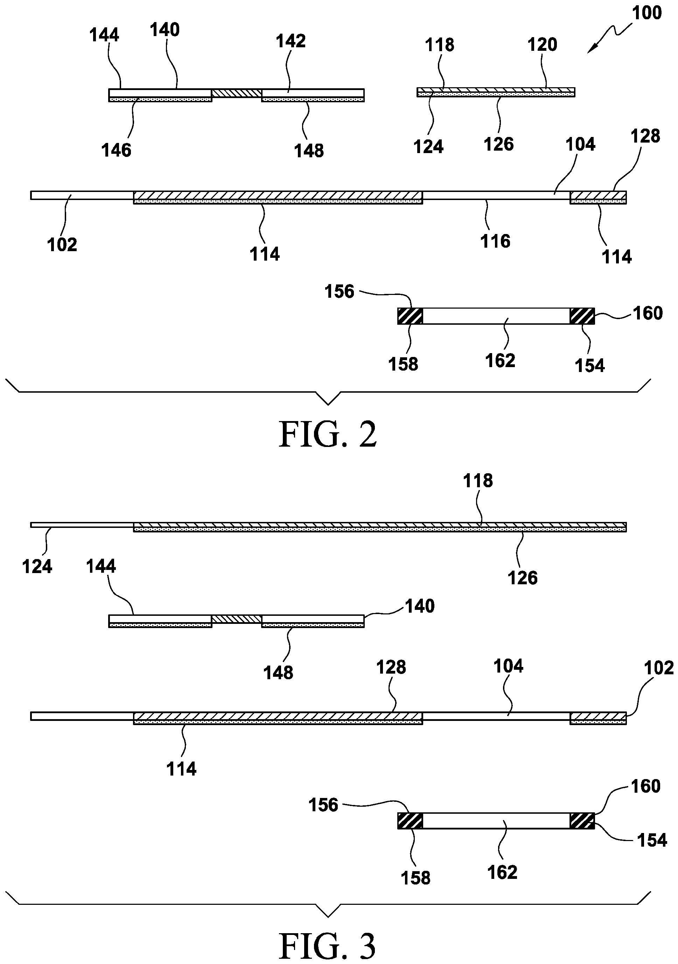

As specifically illustrated in FIG. 2, the dressing may comprise an adhesive layer 114 disposed on a lower, skin-facing surface 116 of the primary layer 102. The adhesive may comprise any suitable medical adhesive. For example, the adhesive may be an acrylate, including methacrylates and epoxy diacrylates. Alternatively, the adhesive may be a silicone based adhesive. Tthe adhesive 114 may be coextensive with the lower surface 116 of the primary layer. Alternatively, the adhesive may extend across only a portion of the lower surface or may be applied in an alternating pattern of adhesive and non-adhesive areas.

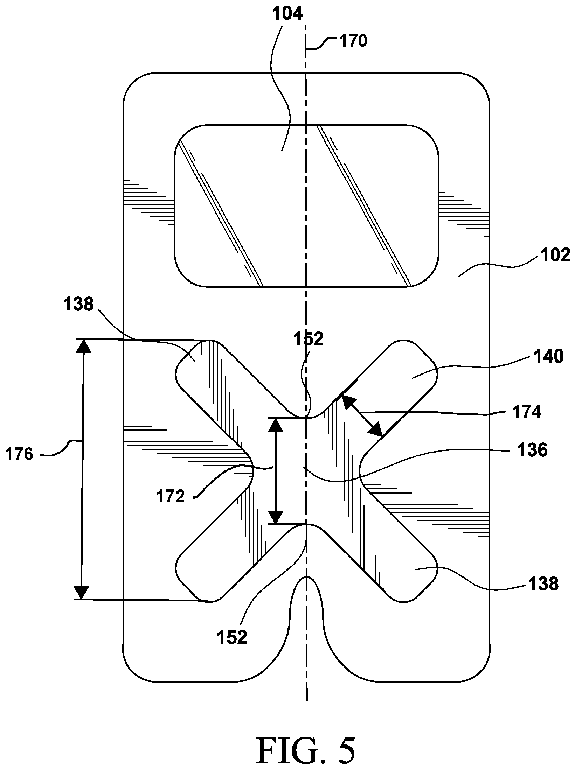

In the dressing 100, shown, for example, in FIGS. 1-2, the primary layer 102 comprises a window 104 positioned within a window portion 106 of the dressing. The window forms an opening that extends through the primary layer forming a hole through the layer 102. The window 104 is positioned within the primary layer such that the window can be positioned around the insertion site 22 when the dressing is applied to a patient. In embodiments of the dressing, the window 104 is positioned around the insertion site, and tubing 24 attached to the catheter 20 is positioned beneath a securement portion 108 of the dressing. In this manner, the catheter site 22 is visible through the window, and the tubing 24 is secured to the patient's skin by the securement portion of the dressing. The window 104 may have a variety of shapes. For example, the window may have a generally domed shape as illustrated in FIG. 1. Alternatively, the window may be rectangular with rounded corners as illustrated in FIG. 5. the window may be formed generally in the shape of a circle, half-circle, square, triangle, polygon, crescent, or any other appropriate shape that allows the insertion site 22 to be visible through the window.

A slot 110 may extend inwardly from an edge of the primary layer 102. The slot may be generally U-shaped. Alternatively, the slot may have a rectangular or V-shape. The slot 110 may be disposed in the securement portion 108 of the dressing. The slot 110 provides a location for tubing to exit from underneath the dressing 102 and may help secure the tubing in place. A closure strip 112 may be secured across the slot 110, underneath the tubing 24 and snug against the tubing, for increased securement of the dressing at the tubing exit. The closure strip may be formed from the same material as the dressing 100 and may be provided along with the dressing on the same release liner. Alternatively, the closure strip 112 may be a length of medical tape or another appropriate adhesive strip.

The dressing, as illustrated in FIGS. 1-2 may comprise a support structure 140. The support structure may comprise a structural layer 142 with an upper surface 144 and a lower surface 146. The support structure may further comprise an adhesive layer 148 deposited on the lower, skin-facing side 146 of the structural layer 142. The adhesive 148 may be any suitable medical grade adhesive and may be coextensive with the lower surface 146 of the structural layer 142. Alternatively, the adhesive may extend across only a portion of the lower surface 146 or may be applied in an alternating pattern of adhesive and non-adhesive areas. The adhesive layer 148 allows the support structure 140 to be adhered to an upper surface 128 of the primary layer 102 in the securement portion 108 of the dressing.

The structural layer 142 comprises a material that has a higher stiffness that the primary layer 102. For example, the structural layer may comprise a polyester film. Preferably, the structural layer 142 has a thickness between 0.005 and 0.100 inches, more preferably between 0.005 and 0.030, more preferably between 0.005 and 0.015, or yet more preferably having a thickness of 0.01 inches. The structural layer 142 may be transparent or may have a color, texture or other property to visually distinguish the support structure 140 from the primary layer 102 of the dressing 100. For example, the structural layer may be transparent, translucent or opaque in combination with various levels of coloration, and, in particular, may comprise a blue-colored, translucent polyester film.

As illustrated in FIGS. 1 and 5-8, the support structure 140 may have a variety of shapes and sizes. For example, as shown in FIG. 1, the support structure 140 may be generally X-shaped. The arms of the X-shape may be relatively long and narrow, as illustrated in FIG. 1, or they may be relatively shorter and wider as illustrated in FIG. 5. The corners of the support structure may be sharply defined or rounded as further illustrated comparing FIG. 1 with FIG. 5. The dressing may comprise a longitudinal axis 170.

The support structure may be formed from two or more portions. FIG. 6 shows a support structure formed from two separate, spaced-apart portions 140a, 140b. The portions illustrated in FIG. 6 are portions that extend laterally and are separated longitudinally. However, it is also contemplated that the portions may extend longitudinally and be separated laterally. A characteristic of embodiments of the dressing is that the support structure 140 extends across a centerline 172 of the dressing, and therefore the tubing 24, at a central region 136. Arms 138 of the dressing then extend from the central region 136 in both lateral and longitudinal directions. In some such embodiments, a portion of the arms 138 extends longitudinally past the edge 152 of the central portion 136 along the centerline of the dressing.

The dressing may be substantially symmetrical about the centerline. The support structure 140 may also be substantially symmetrical about the centerline 172 such that the centerline of the dressing corresponds with a centerline of the support structure. The support structure may extend for an uninterrupted tube covering distance 172 along the centerline. The tube covering distance 172 may be greater than a thickness 174 of the arms 138. In further embodiments, the tube covering distance 172 may be less than the overall longitudinal distance 176 over which the support structure extends.

As illustrated for example, in FIG. 6, a tube covering distance 178 may include breaks or gaps 180 in the support structure 140. In some embodiments, the interrupted tube cover distance 178 may be greater that the thickness 174 of the arms 138. In further embodiments, the gap 180 may also be greater than the thickness of the arms 138. The gap 180 may be created by forming the support structure from two or more separate pieces 140a, 140b. Alternatively the gap 180 may be created by forming a hole through a single-piece support structure.

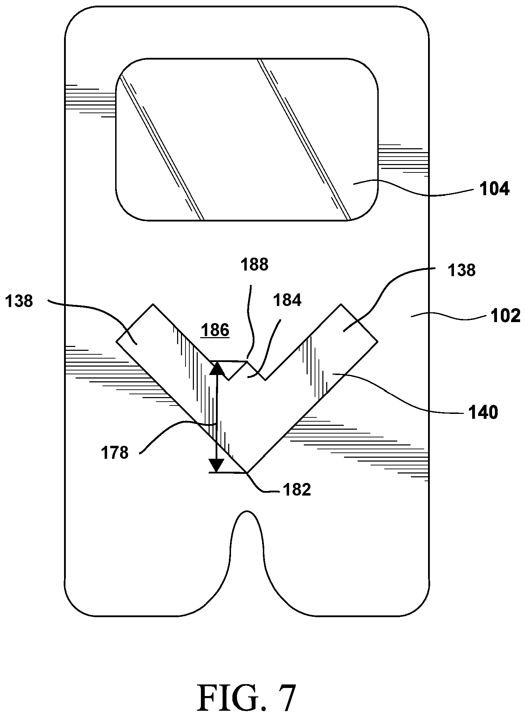

As illustrated for example, in FIG. 7, the support structure 140 may have a generally V-shape. The V-shape may comprise a point 182 that aligns with the centerline of the dressing. The support structure may comprise an extension 184 that extends into the open portion 186 of the V-shape. The extension 184 may increase the tube covering distance 178. The extension 184 may comprise a point 188 that aligns with the centerline of the dressing. The extension 184 may extend in a longitudinal direction for a distance that is shorter than the longitudinal extension of arms 138. Alternatively, the extension may extend for sufficient distance or the arms may be positioned at an angle such that the tube covering distance is greater than the longitudinal extension of the arms.

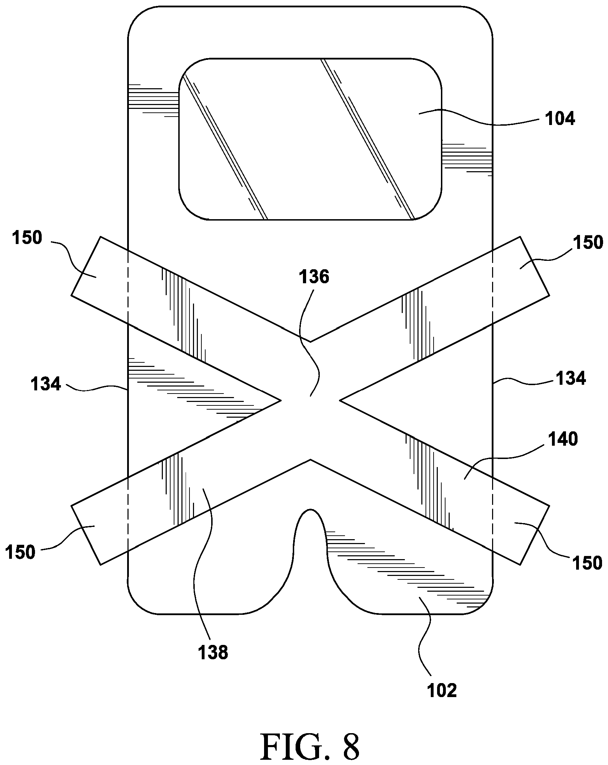

In embodiments, the support structure 140, as illustrated for example in FIG. 8, one or more extensions 150 of the structural layer 142 and/or adhesive layer 148 may extend beyond the securement portion 108 and beyond the periphery 134 of the primary layer 102. The adhesive 148 of the support structure 140 may adhere to top surface 128 of the primary layer 102 within the periphery 134 of the primary layer and may adhere to the patient's skin in the extensions 150 in which the support structure extends beyond the primary layer.

The transparent layer 128 may be coextensive with or smaller than the primary layer 102 such that the extensions 150 of the support structure extend beyond a periphery of the transparent layer. Alternatively, the transparent layer may over the top surface 144 of the support structure 140 in the extension portions 150 such that the transparent layer extends beyond the periphery of the base layer, at least in a portion of the area covered by the support structure extensions 150.

As shown in FIGS. 1-2, the dressing 100 may further comprise a transparent layer 118. The transparent layer may comprise a transparent film 120. The film may have an adhesive layer 126 deposited on the lower, skin-facing side 124 of the transparent film 120. The transparent film 120 may be a polyurethane film, and the adhesive 126 may be any suitable medical grade adhesive. The adhesive 126 may be coextensive with the lower surface 124 of the transparent film 120. Alternatively, the adhesive may extend across only a portion of the lower surface 124 or may be applied in an alternating pattern of adhesive and non-adhesive areas.

The transparent layer 118 may extend across all or a portion of the window portion 106 of the dressing 100, thereby securing the transparent layer 118 to the primary layer 102 and closing the window 104. For example, as illustrated in FIGS. 1- 2, the transparent layer 118 may have generally the same shape as the window 104 except that the transparent layer extends a distance beyond the periphery 132 of the window such that there is an area 130 in which the transparent layer 118 overlaps with a top surface 128 of the primary layer 102. It should be noted that in embodiments of the dressing, the adhesive 126 extends across the portion of the transparent layer 118 that overlaps with the window 104. Because the window is open to the patients skin at the catheter site 22 as well as the catheter 20 and associated tubing 24, the transparent layer may adhere to the catheter 20, tubing 24 and the patients surrounding skin within the window 104.

Alternatively, the transparent layer 118 may extend across all or a portion of the securement portion 108 of the dressing 100 in addition to extending across all or a portion of the window portion 106. The transparent layer 118 may extend across a portion of the support structure 140. For example, as illustrated in FIG. 3, the transparent layer 118 may be coextensive with the primary layer 102. Accordingly, the transparent layer covers the window 104 and covers and is adhered to the top surface 128 of the primary layer 102 as well as the top surface 144 of the support structure 140. The transparent layer may extend over the window 104 and the support structure 140, but have a periphery that is spaced inwardly form the periphery 134 of the primary layer 102 such that a portion of the primary layer top surface 128 adjacent to the primary layer periphery 134 is not covered by the transparent layer 118.

Referring again to FIGS. 2-3, the dressing 100 may further comprise a pad 154. The pad may comprise an upper surface 156 that is adhered to the adhesive layer 114 of the primary layer 102. The pad may further comprise a lower surface 158 that contacts the patients' skin. The pad 154 may have an outer edge 160 and an inner hole or window 162 that is sized to correspond generally to the window 104 in the primary layer.

In this manner, the pad 154 may generally circumscribe the window 104. The pad 154 may be comprise one or more of various materials and may have a thickness that is relatively thinner or thicker than the primary layer. The pad may be formed of absorbent or relatively nonabsorbent material. A characteristic of the pad 154 is that the pad is capable of preventing the egress of certain amounts of exudate and other liquids from beyond the area contained within the pad window 160, that may correspond with the primary layer window 104. The pad may comprise an acrylonitrile butadiene styrene (ABS) foam.

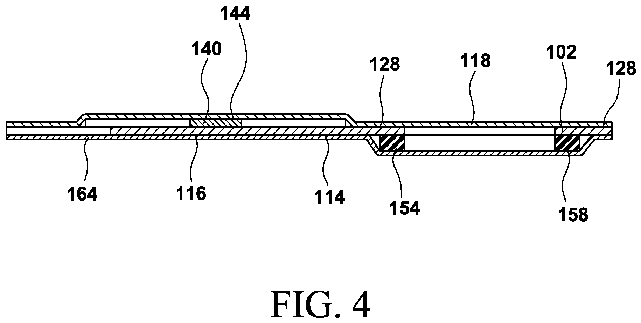

FIG. 4 illustrates the dressing shown in FIG. 3 in an assembled state prior to being applied to a patient. As can be seen, the transparent layer 118 adheres to the top surface 128 of the primary layer 102 at those points were the transparent layer is in contact with the top surface. In addition, the transparent layer 118 adheres to the tip surface 144 of the support structure 140 at those points were the transparent layer is in contact with the top surface. Embodiments of the transparent layer are flexible, and the transparent layer conforms to the surface of the primary layer and support structure to accommodate the thickness of the support structure. As can be seen in FIG. 4, the dressing may further comprise a release liner 164. The release liner may releasably adhere to the lower surface 116 of the primary layer 102 and may extend across the lower surface 158 of the pad 154. The release liner may protect adhesive 114 from prematurely adhering to an undesired location and may be removed from the dressing prior to application on the patient in order to expose the adhesive 114 of the primary layer 102.

While the present invention has been described with reference to one or more particular embodiments, those skilled in the art will recognize that many changes may be made thereto without departing from the scope of the claims. Furthermore, components from one embodiment can be used in other non-exclusive embodiments. Each of these embodiments and obvious variations thereof is contemplated as falling within the scope of the claims.

## Claims

1. A window dressing (100) comprising:
a primary layer (102) having a lower, skin-facing surface (116) and an upper surface (128), the primary layer having a first stiffness and comprising a window portion (106) having a window (104) formed therein and a securement portion (108);
a primary adhesive layer (114) covering at least a portion of the skin-facing surface (116) of the primary layer;
a support structure (140) attached to the upper surface of the primary layer within the securement portion, the support structure comprising:
a structural layer (142) having a lower surface (146) and an upper surface (144), the structural layer having a second stiffness, and
an adhesive layer (148) covering at least a portion of the lower surface of the structural layer and adhering the structural layer to the upper surface of the primary layer;
a transparent layer (118) attached to the upper surface of the primary layer and covering the primary layer, the primary layer window, and at least a portion of the structural layer, the transparent layer comprising:
a transparent film (120) having a lower surface (124) and an upper surface, and
an adhesive layer (126) covering at least a portion of the lower surface of the transparent film and adhering a portion of the transparent layer to the top surface of the primary layer;
**characterized in that** said second stiffness is greater than said first stiffness and **in that** a portion (150) of the support structure extends beyond a periphery of the primary layer (102) and wherein the portion of the support structure (150) extends beyond a periphery of the transparent layer (118).

2. The window dressing of any preceding claim wherein the transparent layer (118) further covers a portion of the securement portion (108) of the primary layer (102).

3. The window dressing of any preceding claim wherein the support structure (140) comprises a central region (136) and an arm (138) extending from the central region in both a longitudinal and a latitudinal direction.

4. The window dressing of any preceding claim wherein the support structure (140) comprises a central region (136) and a plurality of arms (138) extending from the central region in both a longitudinal and a latitudinal direction.

5. The window dressing of claim 4 wherein a first arm of the plurality of arms (138) extends from the central region (136) in a first direction such that an end point of each arm is spaced apart from the central region by a first longitudinal distance and a first latitudinal distance.

6. The window dressing of claim 5 wherein a second arm of the plurality of arms extends from the central region (136) in a second direction such that an end point of each arm is spaced apart from the central region by the first longitudinal distance and the first latitudinal distance.

7. The window dressing of claim 6 wherein the first arm and second arm are symmetrical about a longitudinal axis of the dressing.

8. The window dressing of claim 6 or 7 wherein the first arm and second arm are symmetrical about a latitudinal axis of the dressing.

9. The window dressing of any preceding claim wherein the structural layer (142) of the support structure (140) comprises a polyester film.

10. The window dressing of claim 6 wherein the polyester film has a thickness between 127µm and 762µm.

## Patentansprüche

1. Verband mit Öffnung (100) umfassend:
Eine erste Schicht (102) mit einer unteren zur Haut zeigenden Fläche (116) und einer oberen Fläche (128), die erste Schicht hat eine erste Steifigkeit und umfasst einen Öffnungsabschnitt (106) mit einer Öffnung (104), die darin geformt ist, und einem Befestigungsabschnitt (108);
eine erste Klebschicht (114), die mindestens einen Abschnitt der zur Haut zeigenden Fläche (116) der ersten Schicht bedeckt;
eine Stützstruktur (140), die an der oberen Fläche der ersten Schicht innerhalb des Befestigungsabschnitts angebracht ist, die Stützstruktur umfassend:
Eine Strukturschicht (142) mit einer unteren Fläche (146) und einer oberen Fläche (144), die Strukturschicht hat eine zweite Steifigkeit, und
eine Klebschicht (148), die mindestens einen Abschnitt der unteren Fläche der Strukturschicht bedeckt und die Strukturschicht mit der oberen Fläche der ersten Schicht verklebt;
eine transparente Schicht (118), die an der oberen Fläche der ersten Schicht befestigt ist und die erste Schicht, die Öffnung der ersten Schicht und mindestens einen Abschnitt der Strukturschicht abdeckt, die transparente Schicht umfassend:
Eine transparente Folie (120) mit einer unteren Fläche (124) und einer oberen Fläche, und
eine Klebschicht (126), die mindestens einen Abschnitt der unteren Fläche der transparenten Folie bedeckt und die transparente Schicht mit der oberen Fläche der ersten Schicht verklebt;
**gekennzeichnet dadurch, dass** die zweite Steifigkeit größer ist als die erste Steifigkeit und dass sich ein Abschnitt (150) der Stützstruktur über einen Umfang der ersten Schicht (102) erstreckt und wobei sich der Abschnitt der Stützstruktur (150) über einen Umfang der transparenten Schicht (118) erstreckt.

2. Verband mit Öffnung nach einem der vorhergehenden Ansprüche, wobei die transparente Schicht (118) ferner einen Abschnitt des Befestigungsabschnitts (108) der ersten Schicht (102) bedeckt.

3. Verband mit Öffnung nach einem der vorhergehenden Ansprüche, wobei die Stützstruktur (140) einen
zentralen Bereich (136) und einen Arm (138) umfasst, der sich von dem zentralen Bereich sowohl in Längs- als auch in Breitenrichtung erstreckt.

4. Verband mit Öffnung nach einem der vorhergehenden Ansprüche, wobei die Stützstruktur (140) einen zentralen Bereich (136) und eine Vielzahl von Armen (138) umfasst, die sich von dem zentralen Bereich sowohl in Längs- als auch in Breitenrichtung erstrecken.

5. Verband mit Öffnung nach Anspruch 4, wobei sich ein erster Arm der Vielzahl von Armen (138) von dem zentralen Bereich (136) in eine erste Richtung erstreckt, so dass ein Endpunkt jedes Arms einen Abstand von dem zentralen Bereich in einem ersten Längsabstand und in einem ersten Breitenabstand aufweist.

6. Verband mit Öffnung nach Anspruch 5, wobei sich ein zweiter Arm der Vielzahl von Armen von dem zentralen Bereich (136) in eine zweite Richtung erstreckt, so dass ein Endpunkt jedes Arms einen Abstand von dem zentralen Bereich in dem ersten Längsabstand und dem ersten Breitenabstand aufweist.

7. Verband mit Öffnung nach Anspruch 6, wobei der erste Arm und der zweite Arm symmetrisch um eine Längsachse des Verbands verlaufen.

8. Verband mit Öffnung nach Anspruch 6 oder 7, wobei der erste Arm und der zweite Arm symmetrisch um eine Breitenachse des Verbands verlaufen.

9. Verband mit Öffnung nach einem der vorhergehenden Ansprüche, wobei die Strukturschicht (142) der Stützstruktur (140) eine Polyesterfolie umfasst.

10. Verband mit Öffnung nach Anspruch 6, wobei die Polyesterfolie eine Dicke zwischen 127 µm und 762 µm aufweist.

## Revendications

1. Pansement à fenêtre (100) comprenant :
une couche primaire (102) présentant une surface inférieure faisant face à la peau (116) et une surface supérieure (128), la couche primaire présentant une première rigidité et comprenant une partie de fenêtre (106) présentant une fenêtre (104) formée en son sein et une partie de fixation (108) ;
une couche adhésive primaire (114) recouvrant au moins une partie de la surface faisant face à la peau (116) de la couche primaire :
une structure de support (140) fixée à la surface supérieure de la couche primaire à l'intérieur de la partie de fixation, la structure de support comprenant :
une couche structurale (142) présentant une surface inférieure (146) et une surface supérieure (144), la couche structurale présentant une seconde rigidité, et
une couche adhésive (148) recouvrant au moins une partie de la surface inférieure de la couche structurale et faisant adhérer la couche structurale à la surface supérieure de la couche primaire ;
une couche transparente (118) fixée à la surface supérieure de la couche primaire et recouvrant la couche primaire, la fenêtre de la couche primaire et au moins une partie de la couche structurale, la couche transparente comprenant :
un film transparent (120) présentant une surface inférieure (124) et une surface supérieure, et
une couche adhésive (126) recouvrant au moins une partie de la surface inférieure du film transparent et faisant adhérer une partie de la couche transparente à la surface supérieure de la couche primaire ;
**caractérisé en ce que** ladite seconde rigidité est supérieure à ladite première rigidité et **en ce qu'**une partie (150) de la structure de support s'étend au-delà d'une périphérie de la couche primaire (102) et dans lequel la partie de la structure de support (150) s'étend au-delà d'une périphérie de la couche transparente (118).

2. Pansement à fenêtre selon l'une quelconque des revendications précédentes, dans lequel la couche transparente (118) recouvre en outre une partie de la partie de fixation (108) de la couche primaire (102).

3. Pansement à fenêtre selon une quelconque revendication précédente, dans lequel la structure de support (140) comprend une
région centrale (136) et un bras (138) s'étendant depuis la région centrale dans à la fois une direction longitudinale et une direction latitudinale.

4. Pansement à fenêtre selon une quelconque revendication précédente, dans lequel la structure de support (140) comprend une région centrale (136) et une pluralité de bras (138) s'étendant depuis la région centrale dans à la fois une direction longitudinale et une direction latitudinale.

5. Pansement à fenêtre selon la revendication 4, dans lequel un premier bras de la pluralité de bras (138) s'étend depuis la région centrale (136) dans une première direction de sorte qu'un point d'extrémité de chaque bras est espacé de la région centrale d'une première distance longitudinale et d'une première distance latitudinale.

6. Pansement à fenêtre selon la revendication 5, dans lequel un second bras de la pluralité de bras s'étend depuis la région centrale (136) dans une seconde direction de sorte qu'un point d'extrémité de chaque bras est espacé de la région centrale par la première distance longitudinale et la première distance latitudinale.

7. Pansement à fenêtre selon la revendication 6, dans lequel le premier bras et le second bras sont symétriques autour d'un axe longitudinal du pansement.

8. Pansement à fenêtre selon la revendication 6 ou 7, dans lequel le premier bras et le second bras sont symétriques par rapport à un axe latitudinal du pansement.

9. Pansement à fenêtre selon une quelconque revendication précédente, dans lequel la couche structurale (142) de la structure de support (140) comprend un film de polyester.

10. Pansement à fenêtre selon la revendication 6, dans lequel le film de polyester présente une épaisseur comprise entre 127 µm et 762 µm.
